Europäisches Patentamt

European Patent Office

Office européen des brevets

(11) Veröffentlichungsnummer: **0 154 620 B1**

(12) **EUROPÄISCHE PATENTSCHRIFT**

(45) Veröffentlichungstag der Patentschrift: **31.07.91**

(21) Anmeldenummer: **85890058.2**

(22) Anmeldetag: **11.03.85**

(51) Int. Cl.5: **B01D 71/74**, C08L 89/00, B01D 53/22, B01J 13/02, B01J 47/00, B01D 15/08, A61K 9/64, B65D 65/38

(54) Struktur mit Membranen, die durchgehende Poren aufweisen, Verfahren zum Herstellen dieser Struktur sowie Verwendungen der Struktur.

(30) Priorität: **09.03.84 AT 797/84**

(43) Veröffentlichungstag der Anmeldung:
**11.09.85 Patentblatt 85/37**

(45) Bekanntmachung des Hinweises auf die Patenterteilung:
**31.07.91 Patentblatt 91/31**

(84) Benannte Vertragsstaaten:
**AT BE CH FR GB IT LI NL SE**

(56) Entgegenhaltungen:
**DE-B- 1 237 303**
**DE-B- 1 249 517**
**FR-A- 1 421 584**
**US-A- 3 593 852**

(73) Patentinhaber: **Sleytr, Uwe B., Dipl.-Ing., Dr.**
**Pamhammerplatz 10**
**A-1170 Wien(AT)**

Patentinhaber: **Sara, Margit, Dipl.-Ing.**
**Vorgartenstrasse 90/2/24**
**A-1200 Wien(AT)**

(72) Erfinder: **Sleytr, Uwe B., Dipl.-Ing., Dr.**
**Pamhammerplatz 10**
**A-1170 Wien(AT)**
Erfinder: **Sara, Margit, Dipl.-Ing.**
**Vorgartenstrasse 90/2/24**
**A-1200 Wien(AT)**

(74) Vertreter: **Itze, Peter, Dipl.-Ing. et al**
**Patentanwälte Casati, Wilhelm, Dipl.-Ing. Itze,**
**Peter, Dipl.-Ing. Amerlingstrasse 8**
**A-1061 Wien(AT)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 99, Nr. 1, 04. Juli 1983, Seite 210, Nr. 2009c, Columbus, Ohio, US; D.L. DORSET et al.: "Two-dimensional crystal packing of matrix porin. A channel forming protein in Escherichia coli outer membranes" & J. MOL. BIOL. 1983, 165(4), 701-10

CHEMICAL ABSTRACTS, Band 98, Nr. 17, 25. April 1983, Seite 218, Nr. 139218u, Columbus, Ohio, US; R.M. GARAVITO et al.: "X-ray diffraction analysis of matrix porin, an integral membrane protein from Escherichia coli outer membranes" & J. MOL. BIOL. 1983, 164(2), 313-27

EP 0 154 620 B1

## Beschreibung

Technisches Gebiet

Die Erfindung betrifft eine Struktur, die zumindest eine Membrane mit durchgehenden Poren umfaßt oder aus zumindest einer solchen Membrane gebildet ist, wobei diese Poren insbesondere im Durchmesserbereich von 2 bis 200 nm (nanometer) liegen sollen. Sie betrifft ferner Verfahren zum Herstellen dieser Struktur sowie mehrere vorteilhafte Verwendungen der Struktur.

Stand der Technik

Strukturen mit Membranen, die durchgehende Poren im Durchmesserbereich von 2 bis 200 nm aufweisen, sind z.B. Ultrafiltrationsmembranen, welche in Prozessen zur Fraktionierung oder Konzentrierung von Gemischen aus hochmolekularen organischen Stoffen unterschiedlichen Molekulargewichts verwendet werden. Für industrielle und halbindustrielle Zwecke werden derzeit in vielen Fällen asymmetrische Ultrafiltrationsmembranen eingesetzt, die aus einer sehr dünnen Trennschicht, die für den Stofftransport durch die Membrane sowie für die Selektivität der Trennung maßgeblich ist und deren Dicke im allgemeinen zwischen 100 und 200 nm liegt, und aus einer grobporigen Stützschicht besteht. Die Trennschichten bestehen dabei aus verschiedenen Polymeren, vorzugsweise aus Cellulosederivaten oder Polysulfonen. Solche Ultrafiltrationsmembranen sind entweder Phaseninversionsmembranen oder Composite- Membranen. Bei den Phaseninversionsmembranen wird eine homogene Polymerlösung mit einem Fällungsmittel in Berührung gebracht, wonach an der Kontaktfläche Polymerlösung-Fällungsmittel sich die Membrane ausbildet, in welcher an die feinporige Trennschicht die grobporige Stützschicht anschließt. Bei den Composite-Membranen werden Trenn- und Stützschicht getrennt voneinander hergestellt und erst danach miteinander verbunden.

Bei den bekannten Ultrafiltrationsmembranen ist der Porendurchmesser keine feste Größe, sondern die Durchmesser der Poren variieren gemäß einer statistischen Verteilung um einen Mittelwert. Dieses Verhalten einer Ultrafiltrationsmembrane wird durch ihre Trennkurve charakterisiert. Zur Bestimmung dieser Trennkurve wird für verschiedene ideale Testmoleküle (das sind sphärische Moleküle im ungeladenen Zustand) unterschiedlichen Molekulargewichts (MW) die prozentuale Rückhalterate (R) bestimmt, die bei einer Filtration durch die Ultrafiltrationsmembrane zurückgehalten wird. Die Trennkurve selbst stellt eine Interpolation dieser Meßwerte dar und zeigt die Abhängigkeit dieser prozentualen Rückhalterate vom Logarithmus des Molekulargewichts

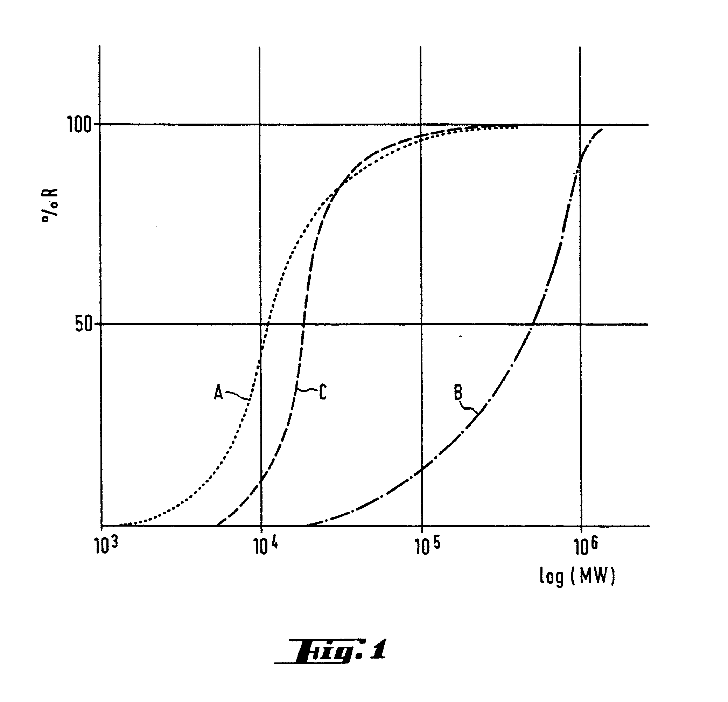

Fig. 1 zeigt ein Diagramm mit den Trennkurven für drei verschiedene im Handel erhältliche Ultrafiltrationsmembranen; nämlich
- Kurve A für die Membrane PSED 25 (Millipore) der Firma Millipore, Bedford Ma, USA,
- Kurve B für die Membrane PSVP 1000 (Millipore) derselben Firma und
- Kurve C für die Membrane PM 30 (Amicon) der Firma Amicon Danvers Ma, USA,

Wie aus diesen Trennkurven deutlich wird, können mit Hilfe dieser Ultrafiltrationsmembranen keine scharfen Trennungen von Molekülen mit geringem unterschiedlichen Molekulargewicht durchgeführt werden.

Eine weitere Kenngröße für die Leistungsfähigkeit einer Ultrafiltrationsmembrane ist die sogenannte Durchflußrate. Das ist die Wassermenge, die pro $m^2$ und Stunde bei einer bestimmten, zwischen den beiden Membranseiten herrschenden Druckdifferenz durch die Membrane hindurchfließt. Bei den bekannten Phaseninversionsmembranen, deren Trennschichten eine Dicke von etwa 100-200 nm aufweisen, setzt die Membrane dem durchfließenden Wasser einen beträchtlichen Widerstand entgegen. Dabei ist die Durchflußrate umso höher umso größer die Anzahl der Poren pro Flächeneinheit der Membranen bzw. umso geringer die wirksame Porentiefe, d.h. die Länge der die Poren bildenden Kanäle ist. Weitere wichtige Qualitätsmerkmale von Ultrafiltrationsmembranen sind ferner ihre chemische und/oder thermische Stabilität.

Darstellung der Erfindung

Der Erfindung liegt die Aufgabe zugrunde, eine Struktur, die zumindest eine Membrane mit durchgehenden Poren umfaßt oder von zumindest einer solchen Membrane gebildet ist, anzugeben, wobei diese Poren insbesondere im Durchmesserbereich von 1 bis 8 nm liegen sollen und wobei bei der Verwendung dieser Struktur als Ultrafiltrationsmembrane man gegebenenfalls scharfe Trennungen zwischen Molekülen mit geringem unterschiedlichen Molekulargewicht realisieren kann; mit welcher Ultrafiltrationsmembrane man ferner eine höhere Durchflußrate erreichen kann als bei den bekannten Ultrafiltrationsmembranen und die eine gute chemische und thermische Stabilität aufweist.

3

Die der Erfindung zugrundeliegende Aufgabe wird in der erfindungsgemäßen Struktur gelöst, die dadurch gekennzeichnet ist, daß die Membrane oder die Membranen, die sich längs ebenen, gekrümmten, zylindrischen oder vesikulären Flächen erstrecken, jeweils aus zumindest einer Schicht von aneinanderliegend miteinander verbundenen und dabei gemäß einem Kristallgitter angeordneten Molekülen, nämlich Proteinmolekülen oder proteinhältigen Molekülen, aufgebaut sind, wobei in diesen Schichten zwischen den Molekülen gemäß einem Gitter angeordnete durchgehende Poren frei bleiben, und daß die Membranen an oder in einem gegebenenfalls porösen Trägerkörper gebunden bzw. eingebunden sind oder daß sie zu einer selbsttragenden Folie vereinigt sind. Dabei sind in diesen Membranen die Proteinmoleküle oder proteinhältigen Moleküle vorteilhaft in einer Einzelschicht oder in mehreren aneinanderliegenden Schichten jeweils gemäß einem Kristallgitter angeordnet miteinander verbunden, wobei in diesen Schichten die aneinanderliegenden Proteinmoleküle oder proteinhältigen Moleküle vorzugsweise durch Nebenvalenzbindungen miteinander verbunden sind.

Nach einer vorteilhaften Ausgestaltung der Erfindung ist die erfindungsgemäße Struktur dadurch gekennzeichnet, daß. mono- oder bifunktionelle Fremdmoleküle an reaktive Gruppen der Proteinmoleküle oder proteinhältigen Moleküle, die vorteilhaft Carboxylgruppen und/oder Aminogruppen und/oder Sulfhydrylgruppen und/oder Hydroxylgruppen sein können, gebunden sind, wobei die Struktur vorteilhaft Membranen mit Schichten aus Proteinmolekülen oder proteinhältigen Molekülen aufweist, innerhalb von denen an in wesentlichen allen diesen Molekülen an denselben reaktiven Stellen Fremdmoleküle gebunden sind.

Gemäß einer weiteren vorteilhaften Ausgestaltung der erfindungsgemäßen Struktur ist sie dadurch gekennzeichnet, daß Proteinmoleküle oder proteinhältige Moleküle der Membranen durch bifunktionelle Fremdmoleküle intramolekular kovalent vernetzt sind und/oder daß sie Membranen aufweist, an denen aneinanderliegende oder benachbarte Proteinmoleküle oder proteinhältige Moleküle, die zur derselben Membran oder zu zwei aneinanderliegenden oder benachbarten Membranen gehören - gegebenenfalls über bifunktionelle Fremdmoleküle - kovalent miteinander vernetzt sind und/oder an denen Proteinmoleküle oder proteinhältige Moleküle - gegebenenfalls durch bifunktionelle Fremdmoleküle - mit dem Trägermaterial vernetzt sind.

In einer noch weiteren vorteilhaften Ausgestaltung der erfindungsgemäßen Struktur ist diese dadurch gekennzeichnet, daß die Fremdmoleküle in den Bereich der zwischen den Proteinmolekülen oder den proteinhältigen Molekülen ausgesparten Membranporen hineinreichen.

Nach einer letzten vorteilhaften Ausgestaltung ist die erfindungsgemäße Struktur gekennzeichnet durch Membranen, deren Proteinmoleküle oder proteinhältigen Moleküle und/oder mit diesen verbundene Fremdmoleküle dissoziierbare Gruppen aufweisen, die unter den Arbeitsbedingungen der Struktur dissoziieren und dadurch vorbestimmte, von diesen Arbeitsbedingungen abhängige elektrische Ladungen annehmen können. Dabei sind diese Membranen, was die Art und/oder Verteilung dieser dissoziierbaren Gruppen an der Membrane anlangt, mit Bezug auf jede zur Membranerstreckung parallele Fläche vorteilhaft asymmetrisch aufgebaut.

Der Erfindung liegt ferner die Aufgabe zugrunde, ein Verfahren zur Herstellung einer Struktur, die zumindest eine Membrane mit durchgehenden Poren umfaßt, insbesondere ein Verfahren zur Herstellung der erfindungsgemäßen Struktur, anzugeben.

Diese Aufgabe wird in dem erfindungsgemäßen Verfahren gelöst, daß dadurch gekennzeichnet ist, daß Proteinmoleküle oder proteinhältige Moleküle, die gegebenenfalls aus Zell-Hüllen, insbesondere aus Zell-Hüllen von Prokaryonten gewonnen wurden, oder Fragmente von Schichten aus solchen Molekülen, die in diesen Schichten jeweils aneinanderliegend miteinander verbunden sind, in einem flüssigen, gegebenenfalls wässrigen Milieu, das gegebenenfalls chaotropen Agenzien, wie Guanidinhydrochlorid oder Harnstoff, und/oder Tenside enthält, in Lösung bzw. in Suspension gebracht werden, daß danach, gegebenenfalls durch Verringerung der Konzentration der chaotropen Agenzien und/oder Tenside und/oder durch Veränderung des pH-Wertes, in dem Milieu Bedingungen geschaffen werden, bei denen die Proteinmoleküle oder proteinhältigen Moleküle und/oder die Schichtfragmente sich dann durch Selbstorganisation zu Membranen verbinden, in welchen die Proteinmoleküle oder die proteinhältigen Moleküle aneinanderliegend gemäß einem Kristallgitter angeordnet sind, in denen zwischen den Molekülen gemäß einem Gitter angeordnete durchgehende Poren frei bleiben, daß die so gebildeten Membranen an bzw. in einem Träger an- bzw. eingebracht werden und daß, gegebenenfalls durch Behandlung mit mono- und/oder bifunktionellen Fremdmolekülen, Proteinmoleküle oder proteinhältige Moleküle der Membranen an ihren reaktiven Gruppen substituiert und/oder über diese reaktiven Gruppen intramolekulär und/oder miteinander und/oder mit dem Träger vernetzt werden. Dabei wird vorteilhaft zum Herstellen der Lösung bzw. Suspension der Proteinmoleküle oder proteinhältigen Moleküle und/oder der aus solchen Molekülen aufgebauten Schichtfragmente eine gegebenenfalls wässrige Suspension aus Zell-Hüllen einer Art hergestellt, die äußere Schichten aufweisen, welche aus aneinanderliegend miteinander verbunden und gemäß einem Kristallgitter angeordneten Proteinmolekülen oder proteinhältigen Molekülen aufgebaut sind, wobei in diesen Schichten zwischen den Molekülen gemäß einem Gitter angeordnete durchgehende Poren frei bleiben, wonach, gegebenenfalls durch Zugabe von chaotropen Agenzien und/oder Tensiden und/oder durch Veränderung des pH-Wertes in das bzw. in dem Milieu, die genannten Proteinmoleküle oder proteinhältigen Moleküle oder Fragmente der aus diesen Molekülen bestehenden Schichten von den Zell-Hüllen abgetrennt werden, und daß die Reste der Zell-Hüllen aus dem Milieu abgetrennt werden.

Nach vorteilhaften Ausgestaltungen des erfindungsgemäßen Verfahrens erfolgt das Abtrennen der Proteinmoleküle oder proteinhältigen Moleküle vorteilhaft durch Erhöhung des pH-Wertes von etwa pH 7.0 auf einen Wert kleiner gleich 13,0, insbesondere aber auf einen Wert kleiner gleich 9,5, oder durch Verringerung des pH-Wertes von etwa pH 7,0 auf einen Wert größer gleich 1,0, insbesondere aber auf einen Wert größer gleich 2,5.

Gemäß einer anderen vorteilhaften Ausgestaltung der Erfindung ist das erfindungsgemäße Verfahren dadurch gekennzeichnet, daß die für die Induzierung der Selbstorganisation der Proteinmoleküle oder proteinhältigen Moleküle und/oder der abgelösten Schichtfragmente zu Membranen durchzuführende Reduktion der Konzentration der chaotropen Agenzien und/oder Tenside und/oder Veränderung des pH-Wertes durch eine Dialyse erfolgt.

In einer weiteren vorteilhaften Ausgestaltung der Erfindung ist das erfindungsgemäße Verfahren dadurch gekennzeichnet, daß die mono- und/oder bifunktionellen Fremdmoleküle Gruppen aufweisen, die mit Carboxylgruppen, Aminogruppen, Sulfhydrylgruppen oder Hydroxylgruppen der Proteinmoleküle oder proteinhältigen Moleküle reagieren.

Nach einer anderen vorteilhaften Ausgestaltung des erfindungsgemäßen Verfahrens erfolgt die Selbstorganisation der Proteinmoleküle oder der proteinhältigen Moleküle und/oder der Schichtfragmente zu Membranen an einer Phasengrenzfläche fest zu flüssig.

Gemäß einer weiteren vorteilhaften Ausgestaltung der Erfindung ist das erfindungsgemäße Verfahren dadurch gekennzeichnet, daß die durch Selbstorganisation der Proteinmoleküle oder proteinhältigen Moleküle und/oder der Schichtfragmente gebildeten Membranen praktisch alle maximale Abmessungen in ihrer Fläche von weniger als 100 $\mu$m, vorzugsweise aber von weniger als 15 $\mu$m ausweisen.

In einer letzten vorteilhaften Ausgestaltung des erfindunsgemäßen Verfahren schließlich erfolgt das An- bzw. Einbringen der Membranen an bzw. in einen porösen Träger durch Anschwemmen an den Träger.

Die Erfindung umfaßt schließlich folgende erfindungsgemäßen Verwendungen der erfindungsgemäßen Struktur oder der nach dem erfindungsgemäßen Verfahren hergestellten Struktur, nämlich

- die Verwendung der Struktur als Ultrafilter, oder als Trennorgan für eine Gasseparation oder als Trennorgan für ein Ionenaustauschverfahren;
- die Verwendung der Struktur als Träger für andere semipermeablen Membranen, die sich über Poren der Membranen der Struktur spannen, wobei gegebenenfalls diese anderen semipermeablen Membranen mit Proteinmolekülen oder proteinhältigen Molekülen der Membranen der Struktur über Carboxylgruppen und/oder Aminogruppen und/oder Sulfhydrylgruppen und/oder Hydroxylgruppen direkt oder über bifunktionelle Fremdmoleküle vernetzt sind. Dabei können diese anderen semipermeablen Membranen vorteilhaft sein: Hyperfiltrationsmembranen, gegebenenfalls Tensid- oder tensidartige Lipoid-Hyperfiltrationsmembranen, oder Trennorgane für eine Gasseparation oder Trennorgane für ein Ionenaustauschverfahren oder Trennorgane für ein Pervaporationsverfahren oder Lösungsdiffusionsmembranen;
- die Verwendung als Trennsäule für eine Durchdringungschromatographie, in der die Membranen gegebenenfalls als Vesikel geformt sind.
- die Verwendung als Hüllenmaterial für Stoffe, wobei das Hüllenmaterial gegebenenfalls als biologisch abbaubares Verpackungsmaterial oder als Kapselhüllen für oral zu verabreichende pharmazeutische Präparate verwendet werden kann.

Beschreibung der erfindungswesentlichen Zeichnungen sowie einiger vorteilhafter Wege zur Ausführung der Erfindung

Die Zell-Hüllen von manchen Prokaryonten, insbesondere von manchen Bazillen weisen eine äußerste Schicht auf, deren elektronenmikroskopisch ermittelte Massenverteilung eine Periodizität aufweist, welche auf eine kristalline Struktur der Schicht schließen läßt. Diese äußerste Schicht, die in der Folge als S-Schicht (S-layer = surface layer) bezeichnet wird, kann von der darunterliegenden peptidoklykanhältigen Schicht der Zell-Hüllen in einem wässrigen Milieu durch Zugabe von chaotropen Agenzien abgetrennt und in Lösung gebracht werden. Wie man durch biochemische Methoden feststellen kann, bestehen diese S-Schichten in den meisten Fällen aus identischen Molekülen, nämlich Proteinmolekülen oder proteinhältigen

EP 0 154 620 B1

Molekülen. Vermindert man nun z.B. durch eine Dialyse die Konzentration dieser chaotropen Agenzien in der Lösung, so bilden sich aus diesen Molekülen durch Selbstorganisation kleine Membranfragmente mit Abmessungen in der Fläche bis zu etwa 10 bis 15 $\mu$m aus, welche dieselbe Massenverteilung aufweisen wie die ursprüngliche S-Schicht. Diese Membranfragmente werden in der Folge P-Membranen genannt. Da solche P-Membranen ferner bei einer weiteren Konzentrationserhöhung der chaotropen Agenzien wieder zerfallen und bei einer neuerlichen Konzentrationsverminderung sich wieder als P-Membranen ausbilden, geht man davon aus, daß die P-Membranen jeweils aus Schichten von aneinanderliegend miteinander verbundenen und dabei gemäß einem Kristallgitter angeordneten Proteinmolekülen oder proteinhältigen Molekülen aufgebaut sind und daß die reversibel lösbare und wieder herstellbare Verbindung zwischen den Molekülen in den P-Membranen über Nebenvalenzen dieser Moleküle erfolgt.

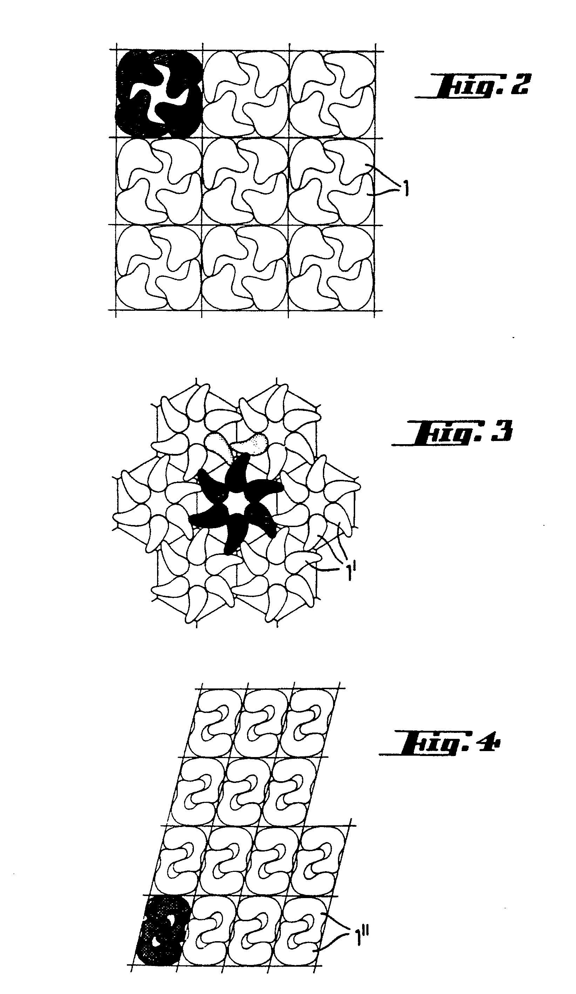

Aus der festgestellten Massenverteilung erkennt man den Gittertyp, der quadratisch, hexagonal oder schräg sein kann. Die Figuren 2 bis 4 zeigen drei Modellvorstellungen, wie man sich die S-Schichten bzw. P-Membranen aus den hier mit 1,1' bzw. 1'' bezeichneten Proteinmolekülen bzw. proteinhältigen Molekülen als Subeinheiten aufgebaut denkt; Fig. 2 zeigt ein quadratisches Gitter mit p4-Symmetrie, Fig. 3 ein hexagonales Gitter mit p6-Symmetrie und Fig. 4 ein schräges Gitter mit p2-Symmetrie. Aufgrund der elektronenmikroskopisch auflösbaren Massenverteilung der S-Schichten bzw. P-Membranen wurde angenommen, daß - wie in den Figuren 2 bis 4 schematisch dargestellt - zwischen den Subeinheiten dieser S-Schichten oder P-Membranen durchgehende Poren bestimmter Form und Größe frei bleiben. Diese Annahme hat sich bestätigt, worauf aber erst weiter unten näher eingegangen werden soll.

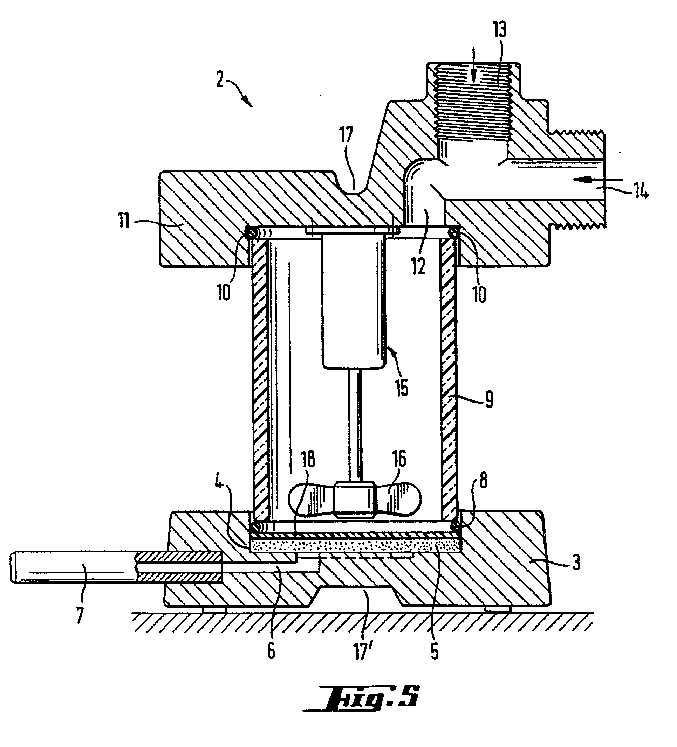

Nachstehend sei nun anhand der Figuren 5 bis 7 in einem ersten Beispiel die Herstellung einer Struktur beschrieben, zu deren Aufbau solche P-Membranen eingesetzt werden und die vorteilhaft als Ultrafilter verwendet werden kann.

Beispiel 1

Bei diesem Beispiel geht man von Zellen des Bacillus stearothermophilus 3c/NRS 1536 aus, dessen Zell-Hüllen aus einer Cytoplasmamembrane, einer peptidoglykanhältigen Schicht und der S-Schicht aufgebaut sind. Die Zellen werden, wie in der Mikrobiologie üblich, zunächst durch Ultraschallbehandlung aufgerissen, die Cytoplasmamembran-Fragmente mit Hilfe von Detergentien desintegriert und die restlichen Zell-Hüllenfragmente durch Waschen von Zellinhaltsstoffen gereinigt. In einem wässrigen Milieu werden dann die S-Schichten durch Zugabe von 5M Guanidinhydrochlorid als chaotropes Agens von der peptidoglykanhältigen Schicht abgetrennt und in Lösung gebracht. Diese Lösung wird dann von den Peptidoglykan-Fragmenten durch Zentrifugieren getrennt und die klare Lösung gegen eine 10mM CaCl$_2$-enthaltende neutrale Pufferlösung dialysiert. Im Zuge dieser Dialyse, bei der in der Lösung die Konzentration des Guanidinhydrochlorids bis praktisch auf Null verringert und die CaCl$_2$-Konzentration erhöht werden, entstehen durch Selbstorganisation die P-Membranen, welche eine quadratische Gitterstruktur (p4-Symmetrie) mit einer Periodizität von 14 nm aufweisen und deren maximale Abmessungen in der Fläche etwa 15 $\mu$m betragen und die in dem wässrigen Milieu durch Rühren in Suspension gehalten werden.

Zur Herstellung des Ultrafilters wird eine Druckapparatur 2, wie sie in Fig. 5 im Schnitt dargestellt ist, verwendet. Sie besteht aus einem Bodenteil 3, welcher eine zylindrische Vertiefung 4 mit einem gerippten Boden aufweist, in der eine poröse Sinterplatte 5 eingelegt ist; der Raum unterhalb der Sinterplatte 5 ist dabei über einen Auslaßkanal 6 mit einem Auslaßstutzen 7 verbunden. Auf diesem Bodenteil 3 ist über einen O-Dichtungsring 8 ein zylindrischer Wandteil 9 aus Plexiglas aufgesetzt, welcher seinerseits über einen zweiten O-Dichtungsring 10 mit einem Dekkelteil 11 verbunden ist, in welchem ein Zuführungskanal 12 mit Anschlüssen 13, 14 für einen Einfüllstutzen bzw. eine Druckgasquelle vorgesehen ist. An der Unterseite des Dekkelteils 11 ist eine Magnetrührelnrichtung 15 befestigt, die mit ihrem Rührer 16 bis an die untere Kante des zylindrischen Wandteils 9 nach unten reicht. Für den Betrieb der Druckapparatur werden Boden- und Deckelteil durch eine bei 17 und 17' angreifende Klemmvorrichtung zusammengehalten.

Zur Herstellung des Ultrafilters wird nun in die Druckapparatur auf der Sinterplatte 5 ein scheibenförmiges Mikrofilter 18 der Firma Nuclepore, Tübingen, BRD als Trägermaterial für das herzustellende Ultrafilter eingelegt. Dieses Mikrofilter 18 besteht aus einer etwa 10 $\mu$m starken Polycarbonatfolie mit Poren von durchwegs gleicher Größe mit einem Porendurchmesser von 0,1 $\mu$m. Über den Anschluß 13 im Deckelteil 11 wird dann die vorstehend beschriebene P-Membranen-Suspension in einer Menge eingefüllt, daß pro cm$^2$ Fläche des Mikrofilters 25 $\mu$g P-Membranen in der Suspension enthalten sind. Danach wird über den Anschluß 14 als Druckgas Stickstoff mit einem Überdruck von 0.5 10$^5$ Pa eingeleitet, wodurch die flüssige Phase der Suspension durch das Mikrofilter 18 und die poröse Sinterplatte 5 gepreßt und die P-Membranen an das Mikrofilter 18 angeschwemmt werden. Danach wird über den Anschluß 13 3 ml einer 2.0 Vol.%igen

6

Lösung von Glutardialdehyd (in 0,1M Natrium-cacodylatpuffer, pH 7,2) auf die angeschwemmten P-Membranen aufgebracht. Darauf wird neuerlich ein Überdruck von $2.10^5$ Pa erzeugt, welcher bewirkt, daß die Glutardialdehydlösung während 20 min bei 20°C durch die angeschwemmten P-Membranen und das Mikrofilter 18 gedrückt werden. Dabei reagiert der Glutardialdehyd, der an beiden Enden eine Carbonylgruppe aufweist, als bifunktioneller Vernetzer mit zwei $\epsilon$-Aminogruppen des Lysins der proteinhältigen Moleküle der P-Membranen und zwar entweder intramolekular, wenn die beiden $\epsilon$-Aminogruppen von demselben proteinhältigen Molekül stammen, oder intermolekular, wenn die beiden $\epsilon$-Aminogruppen von zwei verschiedenen proteinhältigen Molekülen der P-Membranen herrühren.

Nach mehrmaligem Waschen ist die aus dem Mikrofilter 18 und den angeschwemmten und vernetz-ten P-Membranen bestehende Ultrafiltrationsmembrane dann im wesentlichen fertig und kann der Druckapparatur 2 entnommen werden. Die Druckapparatur 2 mit der so hergestellten Ultrafiltrationsmembrane kann aber auch direkt als Ultrafiltrationseinrichtung verwendet werden.

Zur Aufnahme der Trennkurve wurden bei einem durch eingeleiteten Stickstoff erzeugten Überdruck von $2.10^5$ Pa für eine Reihe von Testmolekülen bei pH-Werten, bei denen die Testmoleküle jeweils elektrisch ungeladen sind, also bei ihrem isoelektrischen Punkt (IEP) mit dieser Ultrafiltrationseinrichtung Filtrationstests durchgeführt. Als Testsubstanzen dienten dabei folgende Proteine:

| Nr. | Protein | Molekulargewicht | IEP |
|---|---|---|---|
| 1 | Myoglobin | 17.000 | 6.6 |
| 2 | Subtilisin | 27.000 | 9.4 |
| 3 | Ovalbumin | 43.000 | 4,6 |
| 4 | Rinderserum Albumin | 67.000 | 4,7 |
| 5 | Ferritin | 440.000 | 4,3 |

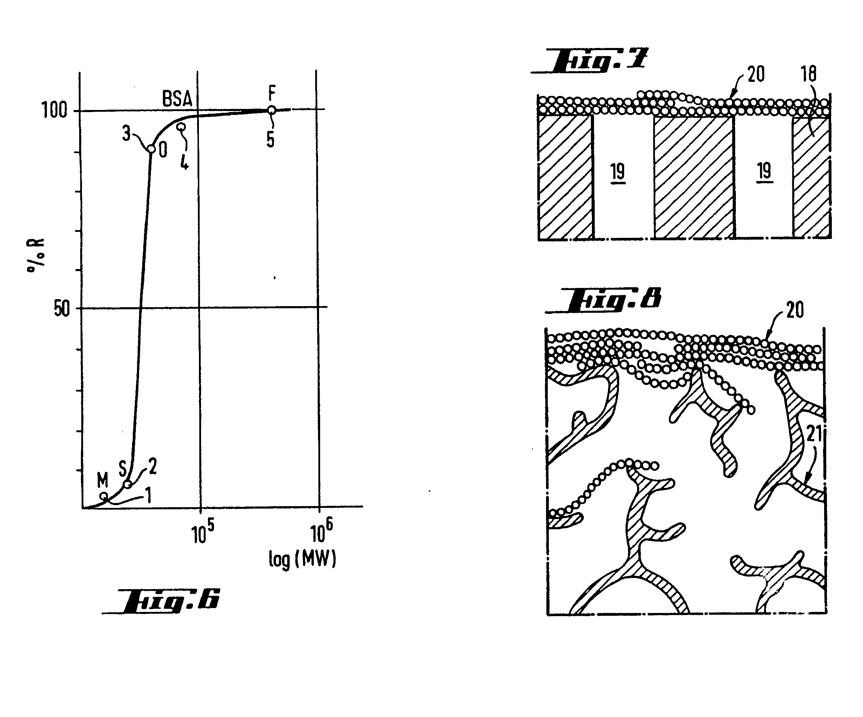

Fig. 6 zeigt ein Diagramm mit der Trennkurve, die aus den Meßwerten für die Rückhaltemengen dieser Testsubstanzen interpoliert wurde. Die Trennkurve zeigt eine scharfe Ausschlußgrenze zwischen den Rückhaltemengen des Subtilisins und des Ovalbumins. Diese Trennkurve beweist auch, daß die P-Membranen tatsächlich durchgehende Poren gleicher Größe aufweisen; der Porendurchmesser wird aufgrund der Form der Trennkurve mit 4-5 nm angenommen.

Die bei dieser Ultrafiltrationsmembrane bei einem Membran-Überdruck von $2.10^5$ Pa bestimmte Durch-flußrate liegt bei 480 1/h.m². Die Durchflußrate ist jedoch von der Menge der angeschwemmten P-Membranen abhängig. So erniedrigt sie sich bei einer angeschwemmten P-Membranmenge von 50 $\mu g/cm^2$ Membranoberfläche auf einen Wert von 220 1/h.m².

Die so hergestellte Ultrafiltrationsmembrane hat noch eine weitere vorteilhafte Eigenschaft die nachstehend näher erläutert werden soll:

Zur elektrischen Nettoladung der S-Schichtfragmente bzw. der unvernetzten P-Membranen in Abhängigkeit des pH-Wertes des sie umgebenden wässrigen Milieus tragen die freien Aminogruppen und Carboxylgruppen in unterschiedlicher Weise bei. Und zwar erzeugen die Aminogruppen bis zu einem pH-Wert kleiner 9.0 positive Ladungen und die Carboxylgruppen im Bereich über pH 2.0 negative Ladungen. Bei einem bestimmten pH-Wert, d.h. an ihrem isoelektrischen Punkt (IEP), kompensieren sich die negativen und positiven Ladungen, so daß die S-Schichtfragmente und P-Membranen dann nach außen als elektrisch neutral erscheinen. Bei dem vorliegenden Beispiel gehen durch die Reaktion des Glutardialdehyds mit denk $\epsilon$-Aminogruppen des Lysins der proteinhältigen Moleküle der P-Membranen potentielle positive Ladungsträger der P-Membranen verloren, wodurch der IEP der vernetzten P-Membranen in den sauren Bereich verschoben wird und bei einem Wert kleiner pH 2.0 liegt. Diese negative Nettoladung der vernetzten P-Membranen ist bei Filtrationen unter physiologischen Bedingungen in vielen Fällen ein wirksamer Schutz gegen ein Verstopfen der Membranporen.

Fig. 7 zeigt in einer Teildarstellung schematisch im Schnitt die gemäß diesem Beispiel hergestellte Ultrafiltrationsmembrane. Auf der Oberfläche des mit durchgehenden Poren 19 versehenen Mikrofilters 18 sind die mit 20 bezeichneten P-Membranen angeschwemmt und durch Vernetzung fixiert. Sie sind dabei in einer Menge aufgebracht, daß die Gesamtoberfläche der P-Membranmenge etwa das zwei- bis dreifache der Fläche der Ultrafiltrationsmembranen ausmacht, so daß die P-Membranen gemittelt etwa in zwei Schichten übereinanderliegen und sich dabei zum Teil überlappen.

EP 0 154 620 B1

Beispiel 2

Abweichend vom Verfahren nach Beispiel 1 geht man bei diesem Beispiel von Zellen des Bacillus stearothermophilus PV 72 aus. Die Zell-Hüllen bestehen auch hier aus einer Cytoplasmamembrane, einer peptidoglykanhältigen Schicht und einer S-Schicht aus proteinhältigen Molekülen. Aus den Zell-Hüllen wird nun - analog wie im Beispiel 1 beschrieben - eine Suspension von P-Membranen hergestellt. Die S-Schicht bzw. die P-Membranen der Zell-Hüllen dieses Bacillus weisen eine hexagonale Gitterstruktur (p6-Symmetrie) mit einer Periodizität von 18 nm auf.

Zum Herstellen einer als Ultrafiltrationsmembrane verwendbaren Struktur wird als Träger ein scheibenförmiges Mikrofilter aus Nylon vom Typ Ultipor $N_{66}$ T der Firma Pall, Cortland, N.Y., USA einer Stärke von 150 $\mu$m in die Druckappartur 2 eingelegt. Dieses Mikrofilter weist freie Amino- und Carboxylgruppen im Verhältnis 1:1 auf. Ähnlich wie gemäß Beispiel 1 wird die P-Membran-Suspension in einer Menge auf das Mikrofilter aufgebracht, daß pro cm² Mikrofilterfläche 30 $\mu$g P-Membranen in der Suspension enthalten sind, und die P-Membranen durch Aufbringen eines Membran-Überdrucks von 2.10⁵ Pa an bzw. in die schwammartige Struktur des Mikrofilters auf- bzw. eingeschwemmt.

Fig. 8 zeigt in einer Teildarstellung im Schnitt das Mikrofilter 21, welches eine offenporige Schwammstruktur hat, wobei die Größen der freigelassenen Poren eine statistische Verteilung um einen Mittelwert aufweisen, sowie die auf- bzw. eingeschwemmte P-Membranen 20. Danach wird bei einem Überdruck von 2.10⁵ 1 ml einer 0,1%igen Dimethylsuberimidat-Lösung (1M Triäthanolaminpuffer, pH 9.5) während 60 min bei 4°C durch die P-Membranen 20 und das Mikrofilter 21 gedrückt. Dabei reagiert das Dimethylsuberimidat als bifunktioneller Imidoester wie ein Aldehyd vorwiegend mit den $\epsilon$-Aminogruppen des Lysins der proteinhältigen Moleküle der P-Membranen intra- sowie intermolekular sowie mit den Aminogruppen des Nylon-Mikrofiltermaterials. Nach mehrmaligem Waschen ist die Ultrafiltrationsmembran dann gebrauchsfertig.

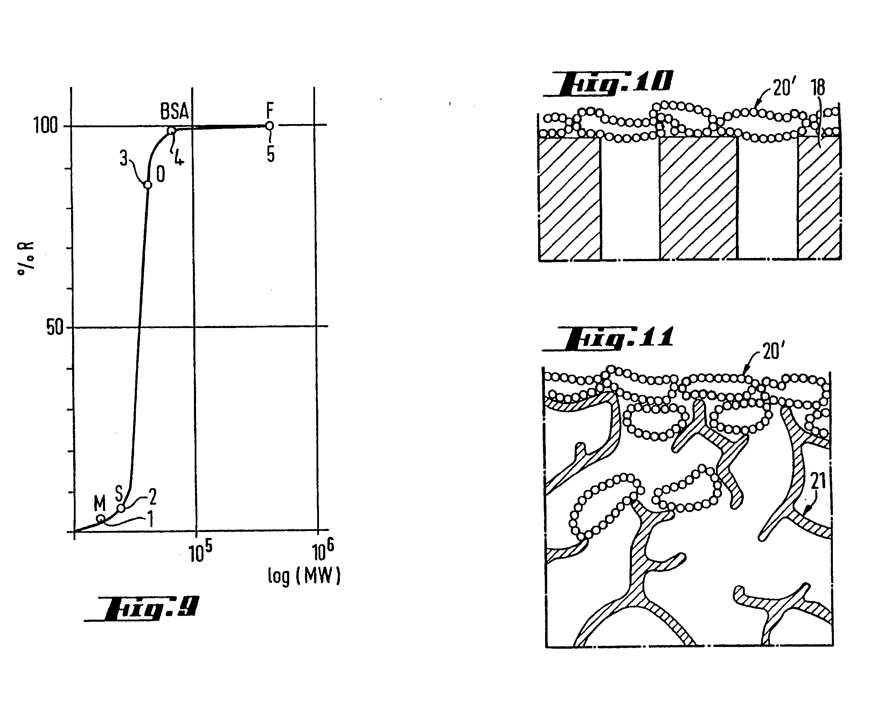

Fig. 9 zeigt das Diagramm mit der Trennkurve der Ultrafiltrationsmembrane. Es zeigt eine ähnlich scharfe Ausschlußgrenze wie die im Beispiel 1 beschriebene Membrane.

Die bei der Reaktion des Dimethylsuberimidats mit den $\epsilon$-Aminogruppen entstehenden Amidine erzeugen in ähnlicher Weise wie die $\epsilon$-Aminogruppen des Lysins eine positive Ladung, so daß die natürliche Nettoladung der P-Membrane durch die Vernetzung kaum geändert wird.

Die in vorstehenden Beispielen 1 und 2 beschriebenen Strukturen mit P-Membranen, die vorteilhaft als Ultrafiltrationsmembranen eingesetzt werden können, haben durch die Vernetzung mit bifunktionellen Fremdmolekülen eine hohe chemische, thermische und mechanische Stabilität. Sie sind insbesondere gegen einen proteolytischen Abbau stabil, autoklavierbar und können auch in einem sauren und alkalischen Milieu (pH 1 bis 13) sowie zusammen mit hochkonzentrierten chaotropen Agenzien (5M Guanidinhydrochlorid, 8M Harnstoff) verwendet werden. Wesentlich ist auch die Resistenz der Strukturen gegen organische Flüssigkeiten, wie Ketone, Alkohole und chlorierte Kohlenwasserstoffe.

Der gewünschte Porendurchmesser der Ultrafiltrationsmembranen wird im wesentlichen durch die Auswahl des zu verwendenden Mikroorganismus erreicht, deren Zeil-Hüllen S-Schichten mit Poren mit in etwa dem angestrebten Porendurchmesser aufweisen. Der gewünschte Porendurchmesser kann dann noch durch Anlagerung von Fremdmolekülen variiert werden, die in den Bereich der Poren der P-Membranen hineinreichen, worauf noch später näher eingegangen werden soll.

P-Membranen die aus einer Molekülschicht aufgebaut sind weisen Schichtdicken von etwa 5 bis 20 nm und Porendurchmesser im Bereich zwischen 1 und 8 nm auf.

Hinsichtlich der Erzeugung der P-Membranen-Suspension sei noch bemerkt, daß man durch Wahl der Konzentration der chaotropen Agenzien bzw. der Tenside erreichen kann, daß die S-Schicht-Fragmente von der peptidoglykanhältigen Schicht der Zell-Hüllen-Fragmente lediglich abgetrennt werden oder daß die S-Schicht-Fragmente selbst desintegriert und in Lösung gebracht werden. Erreicht man z.B. durch Behandeln mit 2,5M Guanidinhydrochlorid nur ein Abtrennen der S-Schicht-Fragmente, wird durch 5M Guanidinhydrochlorid durch Sprengung der Bindungen zwischen den einzelnen Proteinmolekülen oder proteinhältigen Molekülen eine Desintegration der S-Schicht erzielt. Eine Desintegration der S-Schicht kann auch durch eine starke Veränderung des pH-Wertes der die S-Schichten enthaltenen Lösung bewirkt werden; und zwar z.B. durch Absenkung des pH-Wertes von etwa 7,0 auf 2,5, bzw. in manchen Fällen durch Anheben von 7,0 auf 9,5.

Die Flächen der durch Selbstorganisation entstehenden P-Membranen können eben, gekrümmt, zylindrisch oder vesikulär ausgebildet sein. Gemäß den Beispielen 1 und 2 wurden P-Membranen eingesetzt, deren Flächen im wesentlichen eben sind.

Die Figuren 10 und 11 zeigen Varianten der in den Beispielen 1 und 2 beschriebenen Strukturen bei denen die P-Membranen 20' vesikulär ausgebildet sind.

8

EP 0 154 620 B1

Fig. 12 zeigt eine weitere Variante der Struktur gemäß Beispiel 2, bei deren Herstellung vesikulär und eben ausgebildete P-Membranen 20' bzw. 20 eingesetzt wurden. Dabei wurden die vesikulären P-Membranen hauptsächlich in die Poren eingeschwemmt, während man die ebenen P-Membranen überwiegend auf die Oberfläche des Mikrofilters 21 angeschwemmt hat.

Nachstehend seien nun noch einige weitere Beispiele für die Vernetzung der P-Membranen beschrieben.

- Hexamethylendiisocyanat
  reagiert an den P-Membranen bevorzugt mit den Aminogruppen und nach deren Absättigung mit den Hydroxylgruppen, so daß eine intermolekuläre Vernetzung über beide funktionelle Gruppen erfolgen kann. Hexamethylendiisocyanat wird z.B. in einer 1%igen Lösung mit 5% Tetrahydrofuran (Triäthanolaminhydrochlorid, pH 8,0) eingesetzt. Reaktionsdauer ist z.B. 4 Stunden bei 20°C.

- N-N'-Dijodoacetyl-hexamethylendiamin
  greift wie auch andere bifunktionelle Alkylhalide Sulfhydrylgruppen an den P-Membranen an, allerdings unter geeigneten Reaktionsbedingungen auch die Aminogruppen. Im neutralen oder schwach alkalischen Milieu ist dieser Vernetzer jedoch für die Sulfhydrylgruppen spezifisch. Bei der Vernetzung wird das N-N'-Dijodoacetyl-hexamethylendiamin bevorzugt in einer 0,5%igen Lösung (0,1M Natriumacetatpuffer, pH 7,2) verwendet. Die Reaktionsdauer beträgt 3 Stunden bei 4°C.

- 1-Ethyl-3-(3 dimethylaminopropyl)-carbodiimidhydrochlorid (EDC)
  Carbodiimide wie EDC reagieren im sauren Milieu mit den Carboxyl-, Sulfhydryl- und Hydroxylgruppen des Tyrosins an den P-Membranen. Sulfhydrylgruppen müssen - sollen sie an der Reaktion nicht teilnehmen - vorher maskiert werden. Durch die Blockierung der Carboxylgruppen mit EDC wird der pK-Wert der P-Membranen in den sauren Bereich verschoben. 0,1M EDC in aqua dest (0,02M NaOH, pH 8,0) wird z.B. während 18 Stunden bei Raumtemperatur reagieren gelassen.

Es seien nun nachstehend einige Beispiele der Anlagerung von Fremdmolekülen an die Proteinmoleküle oder proteinhältigen Moleküle der P-Membranen gegeben, wobei diese Fremdmoleküle gegebenenfalls die Porengröße der vernetzten P-Membranen beeinflussen:

- Die auf einen porösen Träger, z.B. ein Mikrofilter angeschwemmten P-Membrane werden mit einer Lösung von polykationisiertem Ferritin (5$\mu$g polykationisiertes Ferritin in 1 ml H$_2$O) überschichtet und 5 min bei 20°C inkubiert. Wie elektronenmikroskopisch festgestellt werden konnte, wird unter diesen Bedingungen an jedes Proteinmolekül oder proteinhältiges Molekül der P-Membranen jeweils ein Ferritinmolekül über elektrostatische Wechselwirkungen gebunden. Durch eine nachfolgende Vernetzung mit Glutardialdehyd analog wie bei dem in Beispiel 1 beschriebenen Verfahren werden die Ferritinmoleküle dann kovalent an die P-Membranen gebunden.

- Die auf einem Träger aufgebrachten P-Membranen werden mit einer 1%igen Lösung von Osmiumtetroxyd überschichtet und 30 min bei 20°C inkubiert. Nach Auswaschen der überschüssigen Lösung kann das in den P-Membranen chemisch gebundene Osmium elektronenmikroskopisch und mit Hilfe der Röntgenmikroanalyse nachgewiesen werden. Darauf erfolgt die Vernetzung der P-Membranen wie gemäß Beispiel 1.

- Die auf einem Träger aufgebrachten P-Membranen werden mit einem bifunktionellen Vernetzungsmittel behandelt, dessen Brückenlänge der Größe des Porendurchmessers der P-Membrane nahekommt. Als bifunktionelle Vernetzungsmittel mit unterschiedlicher Brückenlänge kommen dabei z.B. folgende Substanzen in Betracht:
  Tartryl-di-(glycylazid) (TDGA) : 1,3 nm Brückenlänge
  Tartryl-di-($\epsilon$-Aminocaproylazid) (TDCA) : 2,3 nm Brückenlänge
  Bis-methyl-3,8-diaza-4,7-dioxo-5,6-dihydroxydecanbisimidat (DEBE) : 1,4 nm Brückenlänge
  Bis-methyl-4,9-diaza-5,8-dioxo-6,7-dihydroxydodecanbisimidat (DOBE) : 1,7 nm Brückenlänge

- Die Reaktion von TDGA bzw. TDCA, DEBE oder DOBE (0,01M in aqua dest mit 1M Triäthanolamin, pH 8,0) erfolgt während einer Stunde bei 4°C oder während 30 min bei 20°C; danach kann eine Vernetzung wie gemäß Beispiel 1 nachfolgen.

Beispiel 3

In diesem Beispiel wird eine Variante des erfindungsgemäßen Verfahrens beschrieben, bei der eine Schicht aus P-Membranen erzeugt wird, die über etwas größere Flächenbereiche, die insbesondere Abmessungen bis zu 100 $\mu$m haben können, aus jeweils einer einlagigen P-Membrane bestehen. Dazu wird wie in Beispiel 1 beschrieben, eine Suspension aus P-Membranen erzeugt, die von Zell-Hüllen des Bacillus stearothermophilus 3c/NRS 1536 gewonnen wurde. Die S-Schichten der Zell-Hüllen dieses Bacillus sind an ihren an der Zell-Außenseite liegenden Oberfläche, an welcher der Kohlenhydratrest der diese S-Schichten

9

bildenden proteinhältigen Moleküle (Glykoproteine) exponiert ist, elektrisch neutral, während sie an ihrer anderen Oberfläche eine negative Nettoladung aufweisen. Die genannte P-Membranen-Suspension enthält nun außerdem noch freie proteinhältige Moleküle der S-Schichten in Lösung. Ein Mikrofilter mit besonders glatter Oberfläche, wie er auch gemäß Beispiel 1 verwendet wurde, wird an einer Oberfläche mit einer Lösung von Alcianblau (0,1% in aqua dest) behandelt und getrocknet. Das Alcianblau erzeugt in neutralem Milieu auf der Mikrofilteroberfläche eine positive Flächenladung. Die P-Membranen-Suspension wird nun unter leichtem Rühren auf das Mikrofilter aufgebracht. Dabei tritt - induziert durch die positive Flächenladung einerseits an einigen Bereichen der Mikrofilteroberfläche eine Selbstorganisation der sich noch in Lösung befindlichen Proteinmoleküle oder proteinhältigen Moleküle zu P-Membranen ein, wobei die negativ geladene Seite der P-Membranen an der positiv geladenen Mikrofilteroberfläche anliegt, während, andererseits, sich die in Suspension befindlichen P-Membranen, die gewöhnlich maximale Abmessungen in der Fläche bis zu 15 $\mu$m haben können, mit ihrer negativ geladenen Seite bevorzugt auf die noch freien Bereiche der Mikrofilteroberfläche anlegen. Zur Stabilisierung der so gebildeten P-Membran-Schicht wird sie - analog wie im Beispiel 1 beschrieben - vernetzt.

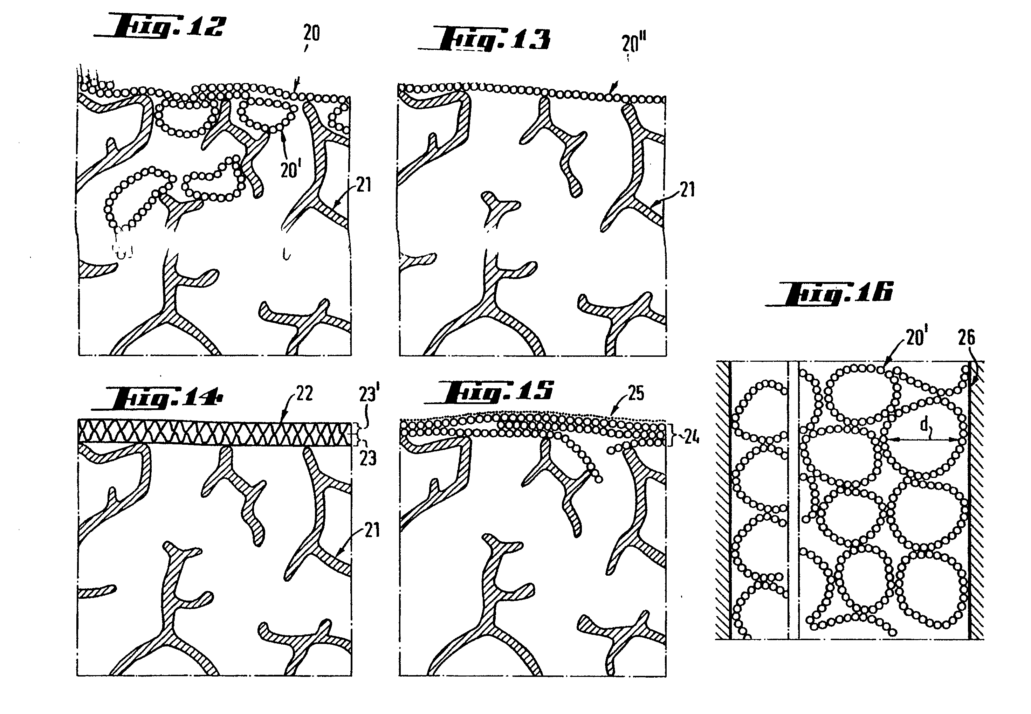

Fig. 13 zeigt in einer Teildarstellung im Schnitt die so erzeugte Struktur, die ebenfalls vorteilhaft als Ultrafiltrationsmembrane verwendet werden kann, mit dem Mikrofilter 21 und einer durch Selbstorganisation an der Phasengrenzfläche fest zu flüssig erzeugten P-Membrane 20'' größerer Flächenausdehnung. Bei ihrer Verwendung als Ultrafiltrationsmembrane weist die so hergestellte Struktur dieselbe Ausschlußgrenze und Trennschärfe auf wie die nach Beispiel 1 hergestellte Struktur.

Ganz allgemein und zum Teil in Abweichung von den in den Beispielen 1 bis 3 beschriebenen Verfahren können als Stützflächen, an welchen sich eine P-Membranen-Schicht ausbildet, auch andere Schichten, wie z.B. peptidoglykanhältige Schichten, Pseudomunreinschichten, Lipidschichten, Polymerschichten, Gele und dgl. verwendet werden. Diese Schichten können, wenn sie durchgehende Poren aufweisen, deren Größe über denen der P-Membranen liegt, als bleibender Träger für die P-Membranen-Schichten dienen oder sie können auch Hilfsschichten sein, die man nach Bildung der P-Membranen-Schicht z.B. durch organische Lösungsmittel wieder entfernt. Die von Hilfsschichten getrennten P-Membranen-Schichten können dann, gegebenenfalls nach einer kovalenten Vernetzung, auf einen den Erfordernissen der beabsichtigten Verwendung der erfindungsgemäßen Struktur besser angepaßten endgültigen Träger aufgebracht werden, mit dem sie dann gegebenenfalls ebenfalls kovalent vernetzt werden können.

Die Oberflächeneigenschaften der "Stützfläche", wie ihr hydrophiler oder hydrophober Charakter bzw. die spezifische Nettoladung und die Ladungsverteilung an der "Stützfläche", erlauben - ähnlich wie bei dem Verfahren nach Beispiel 3 - eine orientierte Bindung der P-Membranen bzw. der Proteinmoleküle oder proteinhältigen Moleküle an der "Stützschicht" und begünstigen damit die Ausbildung der P-Membranen-Schicht. Diese Oberflächeneigenschaften können vorteilhaft auch durch ein elektrisches Feld erzeugt werden, das z.B. von einem elektrisch geladenen, als "Stützfläche" dienenden Metallspiegel ausgeht. Diese Oberflächeneigenschaften sollen u.a. so sein, daß die Bindungskraft zwischen der "Stützfläche" und der sich an ihr anlagernden Proteinmoleküle oder proteinhältigen Moleküle genügend schwach ist, daß die auf dieser "Stützfläche" stattfindende Selbstorganisation dieser Moleküle zu P-Membranen nicht behindert wird. Das ist wichtig für die Ausbildung von P-Membranen mit wenig Störungen im Kristallgitter.

Die bisher beschriebenen Beispiele bzw. ihre Varianten befaßten sich alle mit Strukturen mit P-Membranen, bei welchen jeweils die Proteinmoleküle oder proteinhältigen Moleküle in einer Einzelschicht miteinander verbunden sind. Fig. 14 zeigt nun in einer Teildarstellung schematisch im Schnitt eine weitere Variante der erfindungsgemäßen Struktur, bei der auf ein poröser Mikrofilter 21, wie es auch gemäß Beispiel 2 verwendet wurde, eine P-Membranen-Schicht aufgebracht wird, die aus P-Membranen 22 besteht, welche aus zwei Schichten 23, 23' von Proteinmolekülen oder proteinhältigen Molekülen spiegelbildlich aufgebaut ist. Jede dieser beiden Molekül-Schichten 23, 23' ist an seiner Innenseite bzw. an seiner Außenseite unterschiedlich ausgebildet und die beiden Schichten 23, 23' sind so miteinander verbunden, daß sie die energetisch stabilste Lage einnehmen. Die beiden Schichten 23, 23' können außerdem miteinander bzw. die P-Membrane 22 mit dem Mikrofilter 21 kovalent vernetzt sein.

Einige weitere vorteilhafte Verwendungen der erfindungsgemäßen Struktur, die gewerblich bedeutsam sind.

Neben einer Verwendung als Ultrafiltrationsmembrane, kann die erfindungsgemäße Struktur auch vorteilhaft als Trennorgan für eine Gasseparation oder als Trennorgan für ein Ionenaustauschverfahren verwendet werden.

In weiteren vorteilhaften Verwendungen dient die erfindungsgemäße Struktur als Träger für andere semipermeablen Membranen, die sich über die Poren der P-Membranen der Struktur spannen. Diese anderen semipermeablen Membranen können gegebenenfalls Hyperfiltrationsmembranen, insbesondere mono- oder bimolekulare Hyperfiltrationsmembranen sein. Solche Hyperfiltrationsmembranen, insbesondere Tensid- oder tensidartige Lipoid-Hyperfiltrationsmembranen haben im allgemeinen nur eine Dicke von 2 bis 6 nm und sind besonders fragil. Hyperfiltrationsmembranen werden insbesondere auf den Gebieten der Meerwasserentsalzung, der Abwasseraufbereitung, der Auftrennung von Gemischen organischer Flüssigkeiten, insbesondere zur Kohlenwasserstofftrennung durch Pervaporation oder zur Trennung optischer Antipoden mittels chiraler Trennschichten verwendet.

Fig. 15 zeigt in einer Teildarstellung im Schnitt eine erfindungsgemäße Struktur, deren Herstellung im Beispiel 2 beschrieben wurde (siehe Fig. 8), auf dessen P-Membranen-Schicht 24 die Hyperfiltrationsmembrane 25 aufgebracht ist. Bei der Verwendung der erfindungsgemäßen Strukturen als Träger von Hyperfiltrationsmembranen wird die Filter- bzw. Trennwirkung im wesentlichen durch die Hyperfiltrationsmembrane bestimmt. Fehler, wie kleine Löcher oder dgl. in der P-Membranen-Schicht 24 sind dabei nicht unbedingt störend. Die vernetzte P-Membran-Schicht eignet sich dabei besonders gut als Träger für die Hyperfiltrationsmembranen, da sie eine ausreichende mechanische Stabilität aufweist, um Poren und rauhe Oberflächen der herkömmlichen Trägerschichten für Ultrafiltrationsmembranen so zu überspannen oder auszufüllen, daß die fragilen Hyperfiltrationsmembranen, insbesondere quervernetzte Monoschichten lückenlos aufgezogen oder abgeschieden werden können. Ferner sind die P-Membran-Schichten ausreichend dünn und ihre Porendichte genügend groß, um in Kombination mit Hyperfiltrationsmembranen einen genügend hohen Durchsatz zu gewährleisten.

Eine besonders glatte Oberfläche der P-Membranen-Schicht erhält man insbesondere durch folgendes Verfahren. Es wird - analog wie im Beispiel 1 beschrieben - auf einem Polycarbonat-Träger mit sehr glatter Oberfläche eine P-Membranen-Schicht erzeugt und vernetzt. Der Polycarbonat-Träger wird dann in Chloroform aufgelöst, wobei eine zusammenhängende P-Membranen-Schicht einer Dicke von 5-100 nm zurückbleibt, die dann mit ihrer ursprünglichen, sehr glatten Unterseite nach oben auf einen anderen porösen Träger abgesetzt wird. Auf dieser sehr glatten exponierten Oberfläche der P-Membranen-Schicht wird nun die Hyperfiltrationsmembrane abgesezt und gegebenenfalls mit der P-Membranen-Schicht vernetzt.

Verbunde aus einer P-Membranen-Schicht und einer Hyperfiltrationsmembrane kann man vorteilhaft auch so herstellen, daß man auf eine Hyperfiltrationsmembrane, die eine definierte Oberflächennettoladung aufweist, als "Stützfläche" - z.B. analog wie anhand von Beispiel 3 beschrieben - eine P-Membranen-Schicht ausbildet und diese gegebenenfalls mit der Hyperfiltrationsmembrane vernetzt.

Für eine kovalente Vernetzung zwischen den Hyperfiltrationsmembranen und den P-Membranen-Schichten kommen vor allem Reaktionen in Frage, bei denen Carboxyl-, Hydroxyl-, Amino- und Sulfhydrylgruppen beteiligt sind. Bei P-Membranen aus Glykoproteinen und Hyperfiltrationsmembran-Einzelschichten mit Zuckerresten können auch kohlenhydratchemische Reaktionen angewandt werden.

Verbunde aus einer P-Membranen-Schicht und einer Hyperfiltrationsmembrane können ferner selbst als Träger oder "Stützfläche" für weitere Hyperfiltrationsmembranen oder P-Membranen-Schichten dienen. Solche Mehrfachschicht-Verbunde können in der Ebene der Einzelschichten oder auch zwischen den Einzelschichten durch kovalente Bindungen vernetzt sein. Die Ausbildung eines Sandwich-Verbundes bestehend aus zwei Hyperfiltrationsmembranen an beiden Seiten einer P-Membranen-Schicht erlaubt den Einschuß von Fremdmolekülen, wie Enzymen oder Ladungsträgern, welche das Verhalten eines solchen Sandwich-Verbundes wesentlich beeinflussen können.

Die genannten Verbunde bzw. Mehrfachschicht-Verbunde können auch vorteilhaft die Form von geschlossenen Vesikeln haben, bei deren Herstellung man von einem "Startvesikel" bestehend aus einer Hyperfiltrationsmembrane oder aus einer P-Membranen-Schicht ausgehen kann.

In einer weiteren vorteilhaften Verwendung wird die erfindungsgemäße Struktur als Trennsäule für eine Durchdringungschromatographie eingesetzt. Fig. 16 zeigt in einer Teildarstellung schematisch im Schnitt eine solche Chromatographiesäule 26, in der vesikuläre, gegebenenfalls intra- und intermolekular vernetzte P-Membranen 20' mit einem Innendurchmesser d im Bereich von 1 bis 3 $\mu$m eingefüllt sind. Die zu trennenden Substanzen werden dabei an der Säule oben aufgegeben. Nach Durchlaufen und Eluieren der Substanzen kommen die größeren Moleküle am unteren Ende der Chromatographiesäule früher heraus als die kleineren Moleküle, wobei die Chromatographie eine sehr scharfe Fraktionierung im Bereich der Porengröße der P-Membranen aufweist.

Gemäß einer vorteilhaften Variante lassen sich die Durchflußraten durch die Trennsäule dadurch erhöhen, daß die P-Membran-Vesikel 20' miteinander kovalent vernetzt in morphologisch definierte und mechanisch stabile Aggregate zusammengefaßt sind. Zur Herstellung dieser Aggregate wird ein dichtes Pellet (Sediment) von P-Membran-Vesikeln in dünner Schicht rasch eingefroren, unter flüssigem Stickstoff zu kleinen Bruchstücken zerrieben und in der Folge in einer Mischung aus Methanol und Glutardialdehyd z.B. bei -80°C gefriersubstituiert, wobei die Vernetzung mit Hilfe des Glutardialdehyds stattfindet. Die gewonnenen Aggregate können nun noch weiter zerkleinert, nach Größenklassen gesichtet und für die Füllung der Trennsäule nur bestimmte Größenklassen der Aggregate verwendet werden. Es können ferner vor oder nach dem Einfüllen in die Säule die Aggregate in Puffer übergeführt werden und bzw. es können an den Aggregaten chemische oder enzymatische Veränderungen durchgeführt werden.

In einer letzten vorteilhaften Verwendung schließlich wird die erfindungsgemäße Struktur als Hüllenmaterial für Stoffe verschiedenster Art verwendet. Dieses Hüllenmaterial kann dabei eine vernetzte P-Membranen-Schicht sein, welche wie weiter oben beschrieben auf einer Hilfsschicht oder "Stützfläche" erzeugt wird, wonach die Hilfsschicht gegebenenfalls entfernt wird. Die so erzeugten Folien können z.B. vorteilhaft als Verpackungsmaterial verwendet werden und haben dabei den Vorteil, daß sie gegebenenfalls biologisch abbaubar sein können, wobei man die Abbaugeschwindigkeit durch die Art und den Grad der kovalenten Vernetzung beeinflussen kann.

P-Membranen-Schichten dieser Art können schließlich auch als Kapselhüllen für oral zu verabreichende pharmazeutische Präparate Verwendung finden, wobei es erst durch den proteolytischen Abbau in bestimmten Abschnitten des Verdauungstraktes zur gewünschten Freisetzung des Inhalts kommt. Durch eine gezielte chemische Veränderung der P-Membranen-Schichten der Kapselhüllen kann ihre Abbaugeschwindigkeit und damit der Zeitpunkt der Freisetzung des Kapselinhaltes bestimmt werden. Dabei kann die Freisetzung des Kapselinhaltes bereits vor Auflösung der P-Membranen-Schicht erfolgen, und zwar durch die Porengröße gesteuert. Darüberhinaus könnten pH-Effekte die Freisetzung induzieren.

## Patentansprüche

1. Struktur, die zumindest eine Membrane mit durchgehenden Poren umfaßt oder aus zumindest einer solchen Membrane gebildet ist, dadurch gekennzeichnet, daß die Membrane oder die Membranen, die sich längs ebenen, gekrümmten, zylindrischen oder vesikulären Flächen erstrecken, jeweils aus zumindest einer Schicht von aneinanderliegend miteinander verbundenen und dabei gemäß einem Kristallgitter angeordneten Molekülen, nämlich Proteinmolekülen oder proteinhältigen Molekülen, aufgebaut sind, wobei in diesen Schichten zwischen den Molekülen gemäß einem Gitter angeordnete durchgehende Poren frei bleiben, und daß die Membranen an oder in einem, gegebenenfalls porösen Trägerkörper gebunden bzw. eingebunden sind oder daß sie zu einer selbsttragenden Folie vereinigt sind.

2. Struktur nach Anspruch 1, gekennzeichnet durch Membranen, in denen die Proteinmoleküle oder proteinhältigen Moleküle gemäß einem Kristallgitter in einer Einzelschicht aneinanderliegend angeordnet miteinander verbunden sind.

3. Struktur nach Anspruch 1, gekennzeichnet durch Membranen, in denen die Proteinmoleküle oder proteinhältigen Moleküle in mehreren aneinanderliegenden Schichten jeweils gemäß einem Kristallgitter angeordnet miteinander verbunden sind.

4. Struktur nach einem der Ansprüche 1 bis 3, dadurch gekennzeichnet, daß in den Schichten die aneinanderliegenden Proteinmoleküle oder proteinhältigen Moleküle durch Nebenvalenzbindungen miteinander verbunden sind.

5. Struktur nach einem der Ansprüche 1 bis 4, dadurch gekennzeichnet, daß mono- oder bifunktionelle Fremdmoleküle an reaktive Gruppen der Proteinmoleküle oder proteinhältigen Moleküle gebunden sind.

6. Struktur nach Anspruch 5, dadurch gekennzeichnet, daß die reaktiven Gruppen Carboxylgruppen und/oder Aminogruppen und/oder Sulfhydrylgruppen und/oder Hydroxylgruppen sind.

7. Struktur nach Anspruch 5 oder 6, gekennzeichnet durch Membranen mit Schichten aus Proteinmolekülen oder proteinhältigen Molekülen, innerhalb von denen an im wesentlichen allen diesen Molekülen an denselben reaktiven Stellen Fremdmoleküle gebunden sind.

8. Struktur nach einem der Ansprüche 5 bis 7, dadurch gekennzeichnet, daß Proteinmoleküle oder proteinhältige Moleküle der Membranen durch bifunktionelle Fremdmoleküle intramolekular kovalent vernetzt sind.

9. Struktur nach einem der Ansprüche 1 bis 8, gekennzeichnet durch Membranen, an denen aneinander-

EP 0 154 620 B1

liegende oder benachbarte Proteinmoleküle oder proteinhältige Moleküle, die zur derselben Membran oder zu zwei aneinanderliegenden oder benachbarten Membranen gehören gegebenenfalls über bifunktionelle Fremdmoleküle kovalent miteinander vernetzt sind.

10. Struktur nach einem der Ansprüche 1 bis 9 gekennzeichnet durch Membranen, an denen Proteinmoleküle oder proteinhältige Moleküle - gegebenenfalls durch bifunktionelle Fremdmoleküle - mit dem Trägermaterial vernetzt sind.

11. Struktur nach einem der Ansprüche 6 bis 10, dadurch gekennzeichnet, daß die Fremdmoleküle in den Bereich der zwischen den Proteinmolekülen oder den proteinhältigen Molekülen ausgesparten Membranporen hineinreichen.

12. Struktur nach einem der Ansprüche 1 bis 11, gekennzeichnet durch Membranen, deren Proteinmoleküle oder proteinhältigen Moleküle und/oder mit diesen verbundene Fremdmoleküle dissoziierbare Gruppen aufweisen, die unter den Arbeitsbedingungen der Struktur dissoziieren und dadurch vorbestimmte, von diesen Arbeitsbedingungen abhängige elektrische Ladungen annehmen können.

13. Struktur nach Anspruch 12, gekennzeichnet durch Membranen, die, was die Art und/oder Verteilung dieser dissoziierbaren Gruppen an der Membran anlangt, mit Bezug auf jede zur Membranerstreckung parallele Fläche asymmetrisch aufgebaut sind.

14. Verfahren zum Herstellen einer Struktur nach einem der Ansprüche 1 bis 13, dadurch gekennzeichnet, daß Proteinmoleküle oder proteinhältige Moleküle, die gegebenenfalls aus Zell-Hüllen, insbesondere aus Zell-Hüllen von Prokaryonten gewonnen wurden, oder Fragmente von Schichten aus solchen Molekülen, die in diesen Schichten jeweils aneinanderliegend miteinander verbunden sind, in einem flüssigen, gegebenenfalls wässrigen Milieu, das gegebenenfalls chaotrope Agenzien, wie Guanidinhydrochlorid oder Harnstoff und/oder Tenside enthält, in Lösung bzw. in Suspension gebracht werden, daß danach, gegebenenfalls durch Verringerung der Konzentration der chaotropen Agenzien und/oder Tenside und/oder durch Veränderung des pH-Wertes, in dem Milieu Bedingungen geschaffen werden, bei denen die Proteinmoleküle oder proteinhältigen Moleküle und/oder die Schichtfragmente sich dann durch Selbstorganisation zu Membranen verbinden, in welchen die Proteinmoleküle oder die proteinhältigen Moleküle aneinanderliegend gemäß einem Kristallgitter angeordnet sind, wobei zwischen den Molekülen gemäß einem Gitter angeordnete durchgehende Poren frei bleiben, daß die so gebildeten Membranen an bzw. in einem Träger an- bzw. eingebracht werden und daß, gegebenenfalls durch Behandlung mit mono- und/oder bifunktionellen Fremdmolekülen, Proteinmoleküle oder proteinhältige Moleküle der Membranen an ihren reaktiven Gruppen substituiert und/oder über diese reaktiven Gruppen intramolekulär und/oder miteinander und/oder mit dem Träger vernetzt werden.

15. Verfahren nach Anspruch 14, dadurch gekennzeichnet, daß zum Herstellen der Lösung bzw. Suspension der Proteinmoleküle oder der proteinhältigen Moleküle und/oder der aus solchen Molekülen aufgebauten Schichtfragmente eine gegebenenfalls wässrige Suspension aus Zell-Hüllen einer Art hergestellt wird, die äußere Schichten aufweisen, welche aus aneinanderliegend miteinander verbundenen und gemäß einem Kristallgitter angeordneten Proteinmolekülen oder proteinhältigen Molekülen aufgebaut sind, wobei in diesen Schichten zwischen den Molekülen gemäß einem Gitter angeordnete durchgehende Poren frei bleiben, daß, gegebenenfalls durch Zugabe von chaotropen Agenzien und/oder Tensiden und/oder durch Veränderung des pH-Wertes in das bzw. in dem Milieu, die genannten Proteinmoleküle oder proteinhältigen Moleküle oder Fragmente der aus diesen Molekülen bestehenden Schichten von den Zell-Hüllen abgetrennt werden und daß die Reste der Zell-Hüllen aus dem Milieu abgetrennt werden.

16. Verfahren nach Anspruch 15, dadurch gekennzeichnet, daß das Abtrennen der Proteinmoleküle oder proteinhältigen Moleküle oder der Fragmente von aus diesen Molekülen bestehenden Schichten durch eine Erhöhung des pH-Wertes von etwa 7,0 auf einen Wert kleiner gleich 13,0, vorzugsweise aber kleiner gleich 9,5 erfolgt.

17. Verfahren nach Anspruch 15, dadurch gekennzeichnet, daß das Abtrennen der Proteinmoleküle oder proteinhältigen Moleküle oder der Fragmente von aus diesen Molekülen bestehenden Schichten durch eine Verringerung des pH-Wertes auf einen Wert größer gleich 1,0, vorzugsweise aber größer gleich

13

EP 0 154 620 B1

2,5 erfolgt.

18. Verfahren nach einem der Ansprüche 14 bis 17, dadurch gekennzeichnet, daß die für die Induzierung der Selbstorganisation der Proteinmoleküle oder proteinhältigen Moleküle und/oder der abgelösten Schichtfragmente zu Membranen durchzuführende Reduktion der Konzentration der chaotropen Agenzien und/oder Tenside und/oder Veränderung des pH-Wertes durch eine Dialyse erfolgt.

19. Verfahren nach einem der Ansprüche 14 bis 18, dadurch gekennzeichnet, daß die mono- und/oder bifunktionellen Fremdmoleküle Gruppen aufweisen, die mit Carboxylgruppen, Aminogruppen, Sulfydrylgruppen oder Hydroxylgruppen der Proteinmoleküle oder proteinhältigen Moleküle reagieren.

20. Verfahren nach einem der Ansprüche 14 bis 19, dadurch gekennzeichnet, daß die Selbstorganisation der Proteinmoleküle oder proteinhältigen Moleküle und/oder der Schichtfragmente zu Membranen an einer Phasengrenze fest zu flüssig erfolgt.

21. Verfahren nach einem der Ansprüche 14 bis 20, dadurch gekennzeichnet, daß die durch Selbstorganisation der Proteinmoleküle oder proteinhältigen Moleküle und/oder der Schichtfragmente gebildeten Membrane praktisch alle maximale Abmessungen in ihrer Fläche von weniger als 100 $\mu$m, vorzugsweise aber von weniger als 15 $\mu$m aufweisen.

22. Verfahren nach einem der Ansprüche 14 bis 21, dadurch gekennzeichnet, daß das An- bzw. Einbringen der Membranen an bzw. in den Träger durch Anschwemmen an einen porösen Träger erfolgt.

23. Verwendung der Struktur nach einem der Ansprüche 1 bis 13 oder einer gemäß einem Verfahren nach einem der Ansprüche 14 bis 22 hergestellten Struktur, dadurch gekennzeichnet, daß sie als Ultrafilter oder als Trennorgan für eine Gasseparation oder als Trennorgan für ein Ionenaustauschverfahren verwendet wird.

24. Verwendung der Struktur nach einem der Ansprüche 1 bis 13 oder einer gemäß einem Verfahren nach einem der Ansprüche 14 bis 22 hergestellten Struktur, dadurch gekennzeichnet, daß sie als Träger für andere semipermeable Membranen, die sich über Poren der Membranen der Struktur spannen, verwendet wird, wobei gegebenenfalls diese anderen semipermeablen Membranen mit Proteinmolekülen oder proteinhältige Molekülen der Membranen der Struktur über Carboxylgruppen und/oder Aminogruppen und/oder Sulfhydrylgruppen und/oder Hydroxylgruppen direkt oder über bifunktionelle Fremdmoleküle vernetzt sind.

25. Verwendung nach Anspruch 24, dadurch gekennzeichnet, daß diese anderen semipermeablen Membranen Hyperfiltrationsmembranen, gegebenenfalls Tensid- oder tensidartige Lipoid-Hyperfiltrationsmembranen, sind.

26. Verwendung nach Anspruch 24, dadurch gekennzeichnet, daß diese anderen semipermeablen Membranen Trennorgane für eine Gasseparation sind.

27. Verwendung nach Anspruch 24, dadurch gekennzeichnet, daß diese anderen semipermeablen Membranen Trennorgane für ein Ionenaustauschverfahren sind.

28. Verwendung nach Anspruch 24, dadurch gekennzeichnet, daß diese anderen semipermeablen Membranen Trennorgane für ein Pervaporationsverfahren sind.

29. Verwendung nach Anspruch 24, dadurch gekennzeichnet, daß diese anderen semipermeablen Membranen Lösungsdiffusionsmembrane sind.

30. Verwendung der Struktur nach einem der Ansprüche 1 bis 13, oder einer gemäß einem Verfahren nach einem der Ansprüche 14 bis 22 hergestellten Struktur, dadurch gekennzeichnet, daß sie als Trennsäule für eine Durchdringungschromatographie, in der die Membranen gegebenenfalls als Vesikel geformt sind, verwendet wird.

31. Verwendung einer Struktur nach einem der Ansprüche 1 bis 13 in Form einer Folie, dadurch

14

gekennzeichnet, daß sie als Hüllenmaterial für Stoffe verwendet wird.

32. Verwendung nach Anspruch 31, dadurch gekennzeichnet, daß die Struktur als biologisch abbaubares Verpackungsmaterial verwendet wird.

33. Verwendung nach Anspruch 31, dadurch gekennzeichnet, daß die Struktur als Kapselhüllen für oral zu verabreichende pharmazeutische Präparate verwendet wird.

## Claims

1. Structure which includes at least one membrane with continuous pores or is constituted by at least one such membrane, characterised in that the membrane or the membranes, which extend along flat, curved, cylindrical or vesicular surfaces, are each composed of at least one layer of adjacent molecules, namely protein molecules or protein-containing molecules, which are connected together and arranged in accordance with a crystal lattice, whereby continuous pores remain free in these layers between the molecules arranged in accordance with a lattice and that the membranes are bonded to or bound in an optionally porous carrier body or that they are joined into a self-supporting film.

2. Structure as claimed in claim 1, characterised by membranes in which the protein molecules or protein-containing molecules are connected together in an individual layer arranged adjacent one another in accordance with a crystal lattice.

3. Structure as claimed in claim 1, characterised by membranes in which the protein molecules or protein-containing molecules are connected together in a plurality of adjacent layers and arranged in each case in accordance with a crystal lattice.

4. Structure as claimed in one of claims 1 to 3, characterised in that the adjacent protein molecules or protein-containing molecules in the layers are connected together by secondary bonds.

5. Structure as claimed in one of claims 1 to 4, characterised in that mono- or bifunctional foreign molecules are bonded to reactive groups of the protein molecules or protein-containing molecules.

6. Structure as claimed in claim 5, characterised in that the reactive groups are carboxyl groups and/or amino groups and/or sulphydryl groups and/or hydroxyl groups.

7. Structure as claimed in claim 5 or 6, characterised by membranes with layers of protein molecules or protein-containing molecules within which foreign molecules are bonded to substantially all these molecules at the same reactive positions.

8. Structure as claimed in one of claims 5 to 7, characterised in that protein molecules or protein-containing molecules of the membranes are intramolecularly, covalently cross-linked by bifunctional foreign molecules.

9. Structure as claimed in one of claims 1 to 8, characterised by membranes to which engaging or adjacent protein molecules or protein-containing molecules, which belong to the same membrane or to two engaging or adjacent membranes, are covalently cross-linked with one another, optionally by bifunctional foreign molecules.

10. Structure as claimed in one of claims 1 to 9, characterised by membranes to which protein molecules or protein-containing molecules are cross-linked with the carrier material - optionally by bifunctional foreign molecules.

11. Structure as claimed in one of claims 6 to 10, characterised in that the foreign molecules extend into the region of the membrane pores left free between the protein molecules or the protein-containing molecules.

12. Structure as claimed in one of claims 1 to 11, characterised by membranes whose protein molecules or protein-containing molecules and/or foreign molecules connected thereto have dissociable groups which dissociate under the operating conditions of the structure and can thereby accept predetermined electrical charges dependent on these operating conditions.

13. Structure as claimed in claim 12, characterised by membranes which, as regards the type and/or distribution of these dissociable groups on the membrane, are constructed asymmetrically with respect to each surface parallel to the extent of the membrane.

14. Method of manufacturing a structure as claimed in one of claims 1 to 13, characterised in that protein molecules or protein-containing molecules, which have optionally been obtained from cell walls, particularly from cell walls of prokaryotes, or fragments of layers of such molecules, which are connected together in these layers adjacent to one another, are brought into solution or into suspension in a liquid, optionally aqueous, medium, which optionally contains chaotropic agents, such as guanidine hydrochloride or urea and/or tensides, that thereafter, optionally by reducing the concentration of the chaotropic agents and/or tensides and/or by changing the pH value, conditions are created in the medium at which the protein molecules or protein-containing molecules and/or the layer fragments combine by self-organisation into membranes in which the protein molecules or the protein-containing molecules are arranged engaging one another in accordance with a crystal lattice, whereby continuous pores arranged in accordance with a lattice remain free between the molecules, that the membranes thus formed are introduced onto or into a carrier and that, optionally by treatment with mono- and/or bifunctional foreign molecules, protein molecules or protein-containing molecules of the membranes are substituted at their reactive groups and/or are cross-linked via these reactive groups intramolecularly and/or with one another and/or with the carrier.

15. Method as claimed in claim 14, characterised in that in order to produce the solution or suspension of protein molecules or the protein-containing molecules and/or the layer fragments composed of such molecules an optionally aqueous suspension of cell walls is produced of a type which have outer layers which are composed of engaging protein-molecules or protein-containing molecules connected together and arranged in accordance with a crystal lattice, whereby continuous pores remain free in these layers between the molecules arranged in accordance with a lattice, that optionally by the addition of chaotropic agents and/or tensides to, and/or by altering the pH value of the medium the said protein molecules or protein-containing molecules or fragments of the layers comprising these molecules are separated from the cell walls and that the remainder of the cell walls are separated from the medium.

16. Method as claimed in claim 15, characterised in that the separation of the protein molecules or protein-containing molecules or of the fragments of layers comprising these molecules is effected by an increase of the pH value from about 7.0 to a value less than or equal to 13.0, but preferably less than or equal to 9.5.

17. Method as claimed in claim 15, characterised in that the separation of the protein molecules or protein-containing molecules or of the fragments of layers comprising these molecules is effected by a reduction of the pH value to a value larger than or equal to 1.0, but preferably larger than or equal to 2.5.

18. Method as claimed in one of claims 14 to 17, characterised in that the reduction of the concentration of the chaotropic agents and/or tensides and/or alteration of the pH value which is to be carried out for the induction of the self-organisation of the protein molecules or protein-containing molecules and/or the stripped layer fragments into membranes is effected by dialysis.

19. Method as claimed in one of claims 14 to 18, characterised in that the mono- and/or bifunctional foreign molecules have groups which react with carboxyl groups, amino groups, sulphydryl groups or hydroxyl groups of the protein molecules or protein-containing molecules.

20. Method as claimed in one of claims 14 to 19, characterised in that the self-organisation of the protein molecules or protein-containing molecules and/or of the layer fragments into membranes occurs at a solid to liquid phase boundary.

21. Method as claimed in one of claims 14 to 20, characterised in that the membranes formed by self-organisation of the protein molecules or protein-containing molecules and/or of the layer fragments practically all have maximum dimensions in their surface of less than 100μm, but preferably of less than 15μm.

22. Method as claimed in one of claims 14 to 21, characterised in that the introduction of the membranes onto or into the carrier is effected by deposition onto a porous carrier.

23. Use of the structure as claimed in one claims 1 to 13 or of a structure produced by a method as claimed in one of claims 14 to 22, characterised in that it is used as an ultrafilter or as a separating element for a gas separation or as a separating element for an ion exchange method.

24. Use of the structure as claimed in one of claims 1 to 13 or of a stucture produced by a method as claimed in one of claims 14 to 22, characterised in that it is used as a carrier for other semi-permeable membranes which extend over pores of the membranes of the structure, whereby optionally these other semi-permeable membranes are cross-linked directly or via bifunctional foreign molecules with protein molecules or protein-containing molecules of the membranes of the structure via carboxyl groups and/or amino groups and/or sulphydryl groups and/or hydroxyl groups.

25. Use as claimed in claim 24, characterised in that these other semi-permeable membranes are hyperfiltration membranes, optionally tenside or tenside-like lipoid hyperfiltration membranes.

26. Use as claimed in claim 24, characterised in that these other semi-permeable membranes are separating elements for gas separation.

27. Use as claimed in claim 24, charaterised in that these other semi-permeable membranes are separating elements for an ion exchange method.

28. Use as claimed in claim 24, characterised in that these other semi-permeable membranes are separating elements for a pervaporation method.

29. Use as claimed in claim 24, characterised in that these other semi-permeable membranes are solution-diffusion membranes.

30. Use of the structure as claimed in one of claims 1 to 13 or of a structure produced by a method as claimed in one of claims 14 to 22, characterised in that it is used as a separating column for penetration chromatography in which the membranes are optionally shaped as vesicles.

31. Use of a structure as claimed in one claims 1 to 13 in the form of a film, characterised in that it is used as wrapping material for substances.

32. Use as claimed in claim 31, characterised in that the structure is used as a biologically decomposable packing material.

33. Use as claimed in claim 31, characterised in that the structure is used as capsule shells for pharmaceutical preparations for oral administration.

**Revendications**

1. Structure comprenant au moins une membrane comportant des pores traversants ou bien constituée d'au moins une telle membrane, caractérisée en ce que la ou les membranes est (ou sont) constituée selon le cas d'au moins une couche de molécules liées les unes aux autres et ordonnées suivant un réseau cristallin, à savoir des molécules de protéine ou des molécules renfermant des protéines, disposées suivant des surfaces longitudinales plates, courbées, cylindriques ou sous forme de vésicules, des pores traversants demeurant libres dans ces couches entre les molécules étant ordonnés suivant un réseau, et en ce que les membranes sont liées ou reliées à ou dans un composé vecteur éventuellement poreux, ou bien sont unies à une feuille autoportante.

2. Structure selon la revendication 1, caractérisée par des membranes dans lesquelles les molécules de protéine ou les molécules renfermant des protéines sont liées intimement entre elles suivant un réseau

17

cristallin disposé en une seule couche.

3. Structure selon la revendication 1, caractérisée par des membranes dans lesquelles les molécules de protéine ou les molécules renfermant des protéines sont liées entre elles suivant plusieurs couches adjacentes ordonnées selon le cas suivant un réseau cristallin.

4. Structure selon l'une des revendications 1 à 3, caractérisée en ce que les molécules de protéine ou les molécules renfermant les protéines, adjacentes les unes aux autres, sont liées entre elles dans les couches par des liaisons de valence secondaire.

5. Structure selon l'une des revendications 1 à 4, caractérisée en ce que des molécules étrangères mono- ou bifonctionnelles sont liées aux groupes réactifs des molécules de protéine ou des molécules renfermant des protéines.

6. Structure selon la revendication 5, caractérisée en ce que les groupes réactifs sont des groupes carboxyles, et/ou des groupes amino et/ou des groupes sulfhydryles et/ou des groupes hydroxyles.

7. Structure selon la revendication 5 ou 6, caractérisée par des membranes avec des couches de molécules de protéine ou des molécules renfermant des protéines, parmi lesquelles des molécules étrangères sont liées à toutes ces molécules aux mêmes sites réactifs.

8. Structure selon l'une des revendications 5 à 7, caractérisée en ce que les molécules de protéine ou les molécules renfermant des protéines des membranes sont réticulées par des molécules étrangères bifonctionnelles de manière intramoléculaire et covalente.

9. Structure selon l'une des revendications 1 à 8, caractérisée par des membranes, auxquelles des molécules de protéine ou des molécules renfermant des protéines, liées les unes aux autres ou adjacentes, sont réticulées le cas échéant par des molécules étrangères bifonctionnelles de manière covalente, qui appartiennent à la même membrane ou à deux membranes liées ensemble ou adjacentes.

10. Structure selon l'une des revendications 1 à 9, caractérisée par des membranes auxquelles les molécules de protéine ou des molécules renfermant des protéines sont réticulées avec le matériau supporté, le cas échéant par des molécules étrangères bifonctionnelles.

11. Structure selon l'une des revendications 6 à 10, caractérisée en ce que les molécules étrangères sont introduites dans l'espace, entre les molécules de protéine ou des molécules renfermant des protéines, des pores vides des membranes.

12. Structure selon l'une des revendications 1 à 11, caractérisée par des membranes dont les molécules de protéine ou les molécules renfermant des protéines et/ou avec les molécules étrangères liées à celles-ci possèdent des groupes dissociables, qui dans les conditions d'utilisation de la structure se dissocient et sont susceptibles d'accepter des charges électriques de manière prédéterminée indépendamment de ses conditions d'utilisation.

13. Structure selon la revendication 12, caractérisée par des membranes qui sont construites asymétriquement selon des surfaces parallèles relativement au prolongement de la membrane, ce qui conditionne la place et/ou la répartition de ces groupes dissociables sur la membrane.

14. Procédé de préparation d'une structure selon l'une des revendications 1 à 13, caractérisé en ce qu'on met en solution ou en suspension les molécules de protéine ou les molécules renfermant des protéines, obtenues le cas échéant à partir de parois cellulaires, en particulier de parois cellulaires de procariotes, ou des fragments de couches de telles molécules, qui sont liées les unes aux autres dans ces couches, dans un milieu liquide, le cas échéant aqueux, renfermant le cas échéant des agents chaotropes, tels le chlorhydrate de guanidine ou l'urée et/ou des tenseurs, en ce qu'ensuite, le cas échéant par diminution de la concentration des agents chaotropes et/ou des tenseurs et/ou par modification de la valeur du pH, on maintient les conditions dans le milieu, pour lesquelles les molécules de protéine ou des molécules renfermant des protéines et/ou des fragments de couches sont reliés aux membranes par une organisation propre, dans lesquelles les molécules de protéine ou les molécules renfermant des protéines sont ordonnées les unes à la suite des autres suivant un réseau cristallin, des pores traversants demeurant libres entre les molécules ordonnées suivant un réseau, en ce que les membranes ainsi formées sont amenées sur un support, et en ce que, le cas échéant par traitement avec des molécules étrangères mono- et/ou bifonctionnelles, on substitue les molécules de protéine ou les molécules renfermant des protéines des membranes au niveau des groupes réactifs et/ou on réticule par l'intermédiaire de ces groupes réactifs au niveau intramoléculaire et/ou ensemble et/ou avec le support.

15. Procédé selon la revendication 14, caractérisé en ce que, pour fabriquer la solution ou la suspension de molécules de protéine ou des molécules renfermant des protéines et/ou des fragments de couche obtenus à partir de telles molécules, on prépare une suspension le cas échéant aqueuse de parois cellulaires de manière à obtenir les couches externes, lesquelles sont constituées de molécules de protéine ou de molécules renfermant des protéines liées les unes aux autres et ordonnées selon un réseau cristallin, des pores traversants demeurant libres et ordonnés dans ces couches entre les molécules selon un réseau cristallin, en ce que le cas échéant par addition d'agents chaotropes et/ou des tenseurs et/ou par modification de la valeur du pH dans le milieu on sépare les molécules de protéine citées ou les molécules renfermant des protéines ou des fragments des couches constituées de ces molécules des parois cellulaires et en ce qu'on sépare les restes de parois cellulaires du milieu.

16. Procédé selon la revendication 15, caractérisé en ce que la séparation des molécules de protéine, on des molécules renfermant des protéines, ou des fragments des couches constituées de ces molécules est réalisée par une élévation de la valeur du pH d'une valeur de 7 à une valeur légèrement inférieure à 13, avantageusement inférieure à 9,5.

17. Procédé selon la revendication 15, caractérisé en ce que la séparation des molécules de protéine, ou des molécules renfermant des protéines, ou des fragments des couches constituées de ces molécules est réalisée par une diminution de la valeur du pH à une valeur supérieure à 1,0, avantageusement alors supérieure à 2,5.

18. Procédé selon l'une des revendications 14 à 17, caractérisé en ce que la réduction de la concentration des agents chaotropes et/ou des tenseurs et/ou la modification de la valeur du pH est obtenue par dialyse conduisant aux membranes, pour l'induction de l'auto-organisation des molécules de protéine ou des molécules renfermant des protéines et/ou des fragments de couches non détachés.

19. Procédé selon l'une des revendications 14 à 18, caractérisé en ce que les molécules étrangères mono et/ou bifonctionnelles renferment des groupes qui réagissent avec des groupes carboxyles, amino, sulfhydryles ou hydroxyles des molécules de protéine ou des molécules renfermant des protéines.

20. Procédé selon l'une des revendications 14 à 19, caractérisé en ce que l'autoorganisation des molécules de protéine ou des molécules renfermant des protéines et/ou des fragments de couches en membrane résulte en une phase limite solide à liquide.

21. Procédé selon l'une des revendications 14 à 20, caractérisé en ce que les membranes formées par l'autoorganisation des molécules de protéine ou des molécules renfermant des protéines et/ou des fragments de couches possède en tout point pratiquement une dimension maximale de moins de 100 $\mu$m, avantageusement moins de 15 $\mu$m.

22. Procédé selon l'une des revendications 14 à 21, caractérisé en ce que la fixation ou l'introduction de la membrane sur ou dans le support est obtenue par dépôt sur un support poreux.

23. Utilisation de la structure selon l'une des revendications 1 à 13, ou bien d'une structure préparée selon un procédé de l'une des revendications 14 à 22, caractérisée en ce qu'elle est utilisée comme filtre d'ultrafiltration dans la séparation des gaz ou bien comme moyen de séparation dans un procédé d'échange d'ions.

24. Utilisation de la structure selon l'une des revendications 1 à 13, ou d'une structure préparée selon un procédé de l'une des revendications 14 à 22, caractérisée en ce qu'elle est utilisée comme support pour d'autres membranes semi-perméables, attachées aux pores des membranes de la structure, ou le cas échéant, ces autres membranes semi-perméables sont réticulées avec des molécules de protéine ou des molécules renfermant des protéines des membranes de la structure, directement par des groupes carboxyles, et/ou amino, et/ou sulfhydryles, et/ou hydroxyles ou par des molécules étrangères bifonctionnelles.

25. Utilisation selon la revendication 24, caractérisée en ce que les autres membranes semi-perméables sont des membranes d'hyperfiltration, le cas échéant des membranes d'hyperfiltration tenseur ou du type tenseur.

26. Utilisation selon la revendication 24, caractérisée en ce que les autres membranes semi-perméables constituent des organes séparateurs pour une séparation de gaz.

27. Utilisation selon la revendication 24, caractérisée en ce que ces autres membranes semi-perméables sont des organes séparateurs dans un procédé d'échanges d'ions.

28. Utilisation selon la revendication 24, caractérisée en ce que ces autres membranes semi-perméables sont des organes séparateurs dans un procédé d'évaporation.

29. Utilisation selon la revendication 24, caractérisée en ce que ces autres membranes semi-perméables sont des membranes de diffusion de solution.

30. Utilisation de la structure selon l'une des revendications 1 à 13, ou bien d'une structure préparée selon un procédé suivant une des revendications 14 à 22, caractérisée en ce qu'elle est utilisée sous forme de colonne de séparation pour chromatographie par imprégnation, dans laquelle les membranes se présentent sous la forme, le cas échéant, de vésicules.

31. Utilisation d'une structure selon l'une des revendications 1 à 13, sous forme d'une feuille, caractérisée en ce qu'elle est utilisée comme matériau d'enveloppe pour substance.

32. Utilisation selon la revendication 31, caractérisée en ce que la structure est utilisée comme matériau d'emballage biologiquement dégradable.

33. Utilisation selon la revendication 31, caractérisée en ce que la structure est utilisée sous forme d'enveloppes de capsule de préparations pharmaceutiques administrables oralement.

**Fig. 1**

Fig. 2

Fig. 3

Fig. 4

**Fig. 5**

Fig. 6

Fig. 7

Fig. 8

EP 0 154 620 B1

Fig.9

Fig.10

Fig.11

EP 0 154 620 B1

Fig.12

Fig.13

Fig.14

Fig.15

Fig.16

EP 0 154 620 B1